# EUROPÄISCHE PATENTSCHRIFT

(11) **EP 2 172 163 B1**
(45) Veröffentlichungstag und Bekanntmachung des Hinweises auf die Patenterteilung: **30.11.2016**
(21) Anmeldenummer: 09012539.4
(22) Anmeldetag: 03.10.2009
(51) Int. Cl.: A61B 90/00

(54) **Postoperative Adhäsionsprophylaxe**
Post-operative adhesion prophylaxis
Prophylaxie d'adhésion postopératoire

(30) Priorität: 06.10.2008 DE 102008050122
(43) Veröffentlichungstag der Anmeldung: 07.04.2010
(73) Patentinhaber: Global Medical Consulting Gmbh, 94327 Bogen (DE)
(72) Erfinder: Bertagnoli, Rudolf, 94315 Straubing (DE)

(56) Entgegenhaltungen:
- WO-A1-97/35533
- DE-U1- 20 011 634
- NL-C2- 1 029 010
- US-A- 5 508 036
- US-A- 5 593 441
- US-A1- 2004 215 341
- US-A1- 2008 086 216
- US-A1- 2008 091 277

## Beschreibung

Die Erfindung betrifft eine Adhäsionsprophylaxe bestehend aus einer Membran aus körperverträglichem Material, die zwischen chirurgischer Eingriffstelle und angrenzenden Geweben zur Verhinderung einer Verklebung der Narbenstelle und Gewebe eingelegt wird.

Chirurgische Eingriffe am menschlichen Organismus führen in der Regel im Heilungsprozess zu Vernarbungen, die u.U. angrenzende Gefäße, Organe bzw. Gewebe mit der Vernarbungsstelle verbinden, was dem Patienten nicht nur Beschwerden sondern auch Komplikationen bei einem erneuten Eingriff oder Revision verursachen. Gemäß der DE 196 00 095 und DE 200 11 634 U1 wird dadurch Abhilfe geschaffen, dass nach dem chirurgischen Eingriff eine vom Körper resorbierbare Folie bzw. eine zweischichtige als Beutel gestaltete resorbiere Membran zwischen Eingriffsstelle und angrenzenden Gefäßen, etc. gelegt wird, die eine Verklebung von Geweben mit der Narbe verhindert.

Bei Implantationen oder anderen Eingriffen, die einer Revision bedürfen, könnte eine derartige Einlage u.U. hinderlich aber zumindest nicht hilfreich sein. Z.B. bei der Implantation von Wirbelkörper- oder Bandscheibenprothesen im Lendenbereich vom Bauchraum aus, müssen die vor der Wirbelsäule liegenden Organe und Gefäße verschoben und während der Implantation beiseite gehalten werden. Eine Einlage gemäß dem Stand der Technik wird nach erfolgtem Eingriff zwar eine Verklebung der Organe mit der Wirbelsäule verhindern, aber sie wird bei einem erforderlichen Revisionseingriff keine Erleichterung bringen. Abhilfe hierzu schafft eine nicht resorbierbare, doppelschichtige Membran gemäß WO 97/35533, bei der die beiden Membranen mit einem schwachen Klebstoff verbunden sind, derart, dass die beiden Membranschichten bei einer Revision voneinander getrennt werden können.

Der Erfindung liegt die Aufgabe zugrunde, die Adhäsionsprophylaxe im Hinblick auf Revisionen weiter zu verbessern.

Die Aufgabe wird erfindungsgemäß durch die Merkmale des Anspruchs 1 gelöst.

Gemäß der Erfindung besteht die Membran aus zwei übereinanderliegenden Membranen bzw. Folien gleicher oder unterschiedlicher Anwachseigenschaften. Die beiden Membranen sind an einer Kante verbunden und lassen sich einfach Aufklappen. Damit ist eine optimale Anpassung an die jeweilige Gegebenheit möglich.

Durch Zwischenlegen einer nicht resorbierbaren Membran aus körperverträglichem Material zwischen der Eingriffsstelle und angrenzenden Geweben, Organen, Gefäßen werden nicht nur dem Patienten Beschwerden und Unannehmlichkeiten erspart, sondern damit ist gleichzeitig eine Operationshilfe für einen erneuten chirurgischen Eingriff geschaffen. Die in der Erstoperation eingelegte Membran wird bei Folgeoperationen als Halterung zum Verschieben von Gewebe, wie Organe und Gefäße verwendet, um die Eingriffstelle für den Chirurgen freizulegen.

Die Membran ist dreischichtig ausgebildet, wobei die Zwischenschicht in technisch möglicher Art mit den äußeren Membranschichten lösbar verbunden ist. Es können lösbare Klebverbindungen, Klettverschlüsse verwendet werden.

Im falle eines Revisionseingriffes dient die Zwischenschicht als Trennmittel für die beiden äußeren, den Geweben angrenzenden Membranschichten. Die mehrschichtige Membran ist dabei so ausgestaltet, dass die Zwischenschicht im Körper des Patienten eingesetzten Zustand der Membran von den Membranschichten trennbar bzw. herausziehbar ist, so dass die beiden dadurch getrennten äußeren Membranschichten ohne weiteres voneinander abgehoben bzw. getrennt werden können. Sie dienen in dieser Form zur sauberen Trennung von Geweben und Operationsstelle, die dem Chirurgen nicht nur einen schnellen Zugang zur Eingriffstelle, sondern auch einen einfacheren Eingriff als solches ermöglichen. Dabei ist es unerheblich, ob die Außenseite der beiden Membranschichten mit den angrenzenden Geweben verwachsen sind oder nicht. Diese Ausführung gewährleistet demzufolge eine saubere und sichere Trennung der Membranschichten, um die Eingriffstelle für die Revision rasch freizulegen.

Die Zwischenschicht ist vorzugsweise derart ausgebildet, dass sie eine Flüssigkeitsansammlung oder eine Serumbildung verhindert und damit das Infektionsrisiko reduziert.

Die Membran wird aus bekannten körperverträglichen Materialien, wie Kohlenstofffasern, körperverträglichen Kunststoffen, etc., hergestellt und sie kann vielfältig ausgelegt werden. Je nach Indikation können Membranen mit beidseitig glatten oder beidseitig rauen Oberflächen bzw. mit einer glatten und einer rauen Oberfläche zur Anwendung kommen. Ebenfalls sind doppel- oder mehrschichtige Membranen verwendbar.

Mit der rauen Seite wird ein Verwachsen mit Gewebe zugelassen, während mit einer glatten Seite eine Verklebung dieser Art verhindert werden soll.

Zur sicheren Fixierung der Membran können Stapels verwendet werden, die im Körper auf Dauer erhalten bleiben oder resorbierbar sind. Es ist möglich an Knochenmaterial auf Dauer bestehende Sockel zu verankern, an denen die Membran lösbar, z.B, mittels Schrauben, Haken oder ähnlichen Mittel, verbunden wird. Alternativ wird die Membran durch annähen an Gewebe in ihrer Position gehalten.

Es ist zweckmäßig resorbierbare Stapels zu verwenden, die gegenüber Nähen den operativen Vorgang beschleunigen und die mit der Zeit verschwinden und dem Patienten keine Beschwerden verursachen. Um in diesem Fall ein nachträgliches Verrutschen der Membran zu verhindern, wird diese vorzugsweise mindestens einseitig rau ausgestaltet, um ein Anwachsen an Gewebe zu ermöglichen, wodurch die Lage der Membran stabilisiert wird.

Gemäß einer weiteren Ausgestaltung der Erfindung wird die Membran mit der glatten Seite zur Eingriffsstelle, z.B. Implantatsstelle gerichtet, um eine Verklebung der Membran mit der Operationsstelle bzw. der Prothese zu verhindern und damit eine Beeinträchtigung der Narbstelle bzw. Eingriffstelle zu vermeiden. Über die Raue Seite wird die Membran mit den Organen und ggf. Gefäßen verwachsen. Diese Ausführung hat den weiteren Vorteil, dass im Falle eines Revisions- bzw. erforderlichen erneuten Eingriffs, die vorgelagerten Organe und Gefäße mit der Membran als Gesamtpaket leichter zu Handhaben bzw. zu verschieben sind, um die Eingriffsstelle freizulegen.

Gemäß einer weiteren Ausgestaltung der Erfindung wird die Membran aus einer Kombination von Materialien dergestalt hergestellt, daß die Membran unterschiedliche Eigenschaften auf beiden Seiten hat. Diese unterschiedlichen Eigenschaften können auch mit zwei entsprechend unterschiedlich ausgelegten Membranen realisiert werden, die flächig miteinander verbunden sind. Mit einer derartigen Ausgestaltung der Membran kann eine optimale Anpassungen einerseits zur Narbe und andererseits zu den Geweben, Organen und/oder Gefäßen erreicht werden.

Bei entsprechender Indikation kann die Membran durchaus umgekehrt eingesetzt werden, nämlich mit der glatten Seite zu den Organen und der rauen Seite zur Eingriffstelle gerichtet.

Zur Erzeugung unterschiedlicher Anwachseigenschaften kann die Membran auch einseitig entsprechend beschichtet werden.

Als Zwischenschicht eignet ich auch eine Ringausführung, die nur den Randbereich der beiden äußeren Membranschichten voneinander trennt.

Eine vorteilhafte Ausgestaltung besteht in einer dreischichtigen Membran, deren Äußeren Seiten ein Anwachsen an Gewebe erlauben und deren Zwischenschicht lösbar und wieder einsetzbar ist. Hier bietet sich dem Chirurgen eine sauber definierte Operationsstelle nach dem Trennen bzw. Aufklappen der beiden Membranschichten, indem die Membran die Umgebung der Operationsstelle abdeckt, während für die Eingriffsstelle in der eingriffseitigen Membranschicht eine Öffnung vorgesehen ist, die mit einem lösbaren Fensterdeckel abgedeckt ist.

Die erfindungsgemäße Membran zur Adhäsionsprophylaxe kann überall dort verwendet werden, wo ein Anwachsen zwischen Geweben und Operationsstelle verhindert werden soll und wo ein erneuter Eingriff möglich ist, wie bei Darmkrankheiten, Zystenbildungen, Implantationen, etc.

Gemäß einer weiteren Ausgestaltung ist die Membran Bestandteil eines Zuggurtungsbands oder mit diesem verbunden. Dieses ist bei Rückgrad- oder Bandscheibenoperationen zweckmäßig, bei denen beispielweise ein Zuggurtungsband eingesetzt werden muß. Dabei kann die entlang des Zuggurtungsband verbundene Membran aus jedem Körperverträglichen Material hergestellt sein. Das Zuggurtungsband kann auch direkt durch die Membran gebildet sein.

Die Erfindung wird anhand von in der Zeichnung schematisch dargestellten Beispielen näher beschrieben.
Fig. 1 zeigt den Stand der Technik,
Fig. 2 zeigt ein Ausführungsbeispiel
Fig. 3 und 4 stellen ein Ausführungsbeispiel und dessen Anwendung bei Erst- bzw. Nachoperation dar,
Fig. 4a zeigt einen Ausschnitt aus Fig.4, und
Fig. 5 zeigt ein weiteres Ausführungsbeispiel.

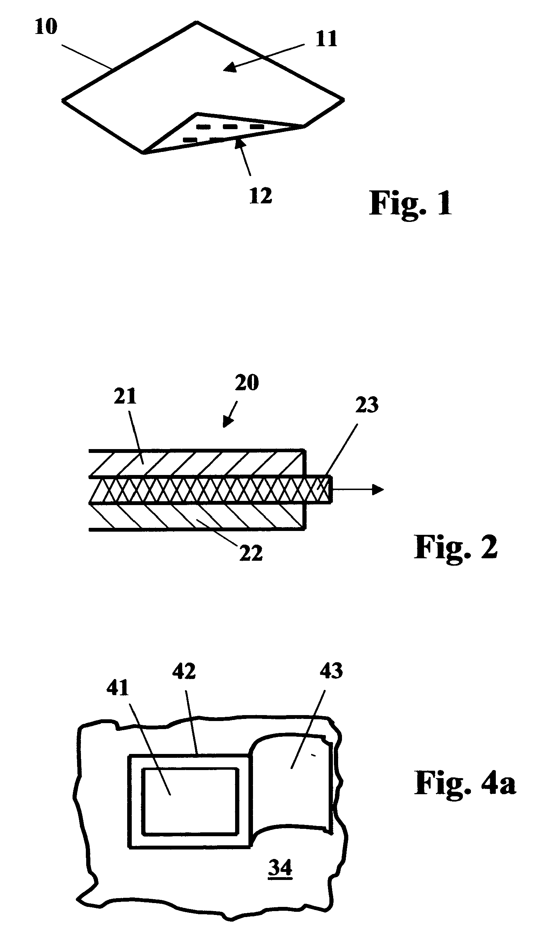

Eine einfache, an sich bekannte Adhäsionsprophylaxe besteht gemäß Fig. 1 aus einer Membran 10 aus einem körperverträglichen Material, die im dargestellten Beispiel eine glatte Seite 11 und eine raue Seite 12 aufweist. Mit dieser Ausführung wird bezweckt, dass die Membran nur einseitig mit Gewebe verwachsen kann. Die ungleichen Eigenschaften können durch entsprechende Auslegung einer einschichtigen oder zweischichtigen Membran oder durch Beschichtung einer bzw. beider Membranseiten erreicht werden.

Je nach Anwendung können auch Membranen mit beidseitig gleichen Eigenschaften, d.h. beide Seiten glatt oder rau, verwendet werden.

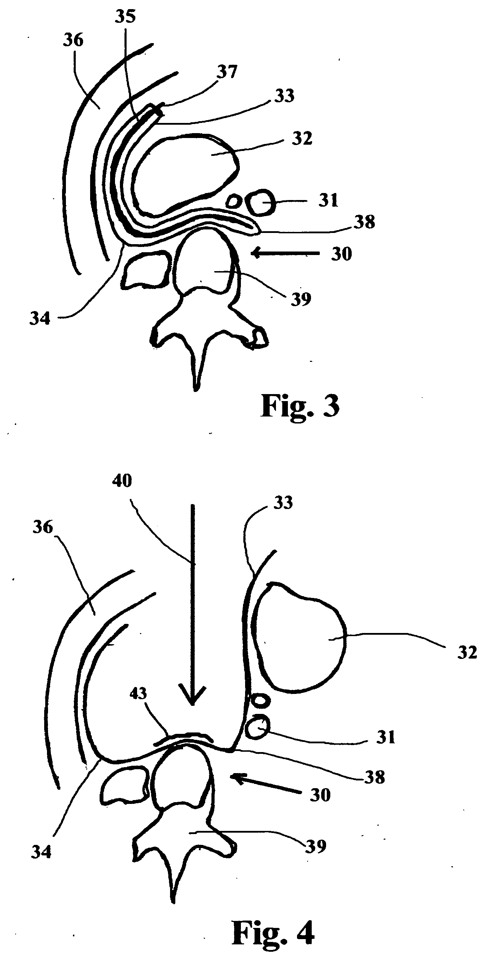

Fig. 2 zeigt ein erfindungsgemäßes Ausführungsneispiel mit einer dreischichtigen Membran 20, wobei eine Zwischenschicht 23 die beiden eigentlichen Membranenschichten 21,22 einerseits trennt und andererseits lösbar verbindet derart, dass die Zwischenschicht 23 bei im Körper des Patienten eingesetzten Position in Pfeilrichtung herausziehbar ist und damit die beiden äußeren Membranschichten 21,22 zum aufklappen freigibt. In der Regel erfolgt die Trennung durch abziehen der oberen Schicht 21 und dann wird die Zwischenschicht 23 entfernt. Die Zwischenschicht ist z.B. eine lösbare Klebschicht, eine über Klettverschluss mit den außenschichten verbindbare Schicht oder dergleichen sein.
Die beiden Membranschichten 21,22 sind an einer Kante miteinander verbunden und lassen sich nach dem Entfernen der Zwischenschicht 23 wie ein Buch aufklappen, wie es in Fig. 4 mit den Membranschichten 33, 34 gezeigt ist. Damit legen sie die Operationsstelle 30 frei, wobei sie gleichzeitig die Gewebestellen 31,32,36,39 (Fig.4) um die Eingriffsstelle verdecken. Für die Eingriffstelle 30 als Solches ist in der eingriffseitigen Membranschicht 34 eine Öffnung 41, Fig.4a, vorgesehen, die mit einem Fensterdeckel 43 lösbar verschlossen werden kann. Dafür ist eine lösbare Klebschicht oder Klettstreifen 42 um die Öffnung 41 vorgesehen.

Mit den Figuren 3,4 und 4a wird die Anwendung der Erfindung an Hand eines Beispieles einer Implantation eines Wirbelkörperersatzes oder einer Bandscheibenprothese beschrieben, die an der Frontalseite durchgeführt wird. Die Figuren 3 und 4 zeigen einen Querschnitt durch den Lendenbereich eines Patienten, wobei Fig. 3 den Zustand nach der ersten Operation und Fig. 4 die Vorbereitung für einen Revisionseingriff darstellen.

Bei einen derartigen Eingriff müssen Gefäße 31 und/oder Organe 32 die vor der Wirbelsäule 39 bzw. der Eingriffstelle 30 liegen beiseite geschoben und gehalten werden.

In der Erstoperation wird nach erfolgter Implantation der Prothese eine Membran 33-35 über die behandelte Rückgradstelle 30 gelegt und entsprechend fixiert, z.B. mittel Stapels und danach werden die Organe 32 und Gefäße wieder zurückgesetzt. Dieser Zustand ist in Fig. 3 dargestellt, bei der die Membran 33-35 zwischen der Operationsstelle 30 und den Gefäßen/Organen 31,32 und zwischen diesen und dem Bauchfell 36 zu liegen kommt. Damit wird verhindert, dass die Organe 32 und Gefäße 31 mit der Vernarbung an der Operationsstelle 30 verwachsen und dem Patienten Beschwerden verursachen. Insbesondere dient die Membran 33-35 im Falle einer erneuten Operation zur wesentlichen Vereinfachung zumindest der Vorbereitung zur eigentlichen Operation. Im Beispiel der Fig.3 und 4 findet eine mehrschichtige Membran, wie sie im Zusammenhang mit Fig. 2 beschrieben wurde, Anwendung. Die Zwischenschicht 35 ist mit den beiden Membranschichten 33,34 lösbar verbunden, mittels lösbarem Klebstoff, Klettverschluss oder dergleichen. Die Zwischenschicht 35 ist derart ausgebildet, dass sie eine Serumbildung zwischen den beiden Membranschichten verhindert, um Infektionsrisiken zu reduzieren.

Beim Revisionseingriff wird mittels dem hervorstehenden Ende 37 der Zwischenschicht 35 die Zwischenlage herausgezogen bzw. je nach Verbindung von den beiden Membranschichten 33,34 getrennt. Nach Entfernung der Zwischenlage 35 lassen sich die beiden Membranschichten 33,34 mühelos voneinander trennen und aufklappen, wobei mit der Membranschicht 33 die Gefäße 31 und Organe 32 als Einheit zur Seite geschoben werden, so dass der Chirurg einen freien Zugang 40 zur Operationsstelle 30 erhält.

Die beiden äußeren Membranschichten 33,34 sind entlang einer Kante 38, eine Faltkante bildend, miteinander verbunden.

Für den Zugang zur Eingriffstelle 30 ist in der unteren Membranschicht 34 eine Öffnung 41, Fig.4a, vorgesehen, die durch Abnehmen des Fensterdeckels 43 freigelegt wird. Damit sind die Vorbereitungen für den eigentlichen Eingriff, z.B. das Auswechseln einer Bandscheibenprothese, beendet.

Nach dem Revisionseingriff, z.B. dem Auswechseln der Bandscheibenprothese, wird der oder ein neuer Fensterdeckel 43 über die Klebstelle 42 gelegt und mit der Membranschicht 34 verbunden, die Zwischenschicht 35 oder eine neue Zwischenschicht wieder eingebracht und mit den Membranschichten 33,34 lösbar verbunden.

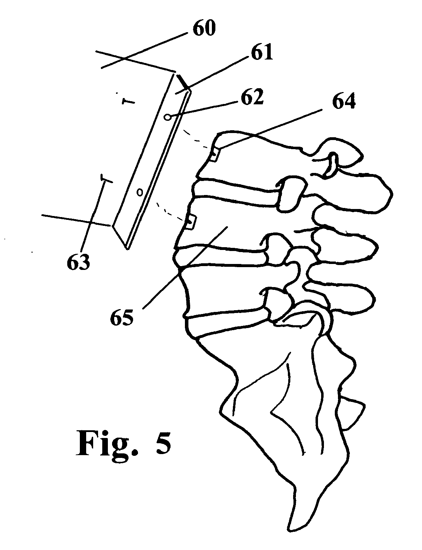

Die Fixierung einer Membran erfolgt, soweit notwendig, je nach Anwendung durch Nähen, mit resorbierbaren oder beständigen Stapels oder mittels an Knochen 65 angebrachten Befestigungsplatten 64, Fig.5 oder dergleichen.

In der Fig.5 ist ein Beispiel gezeigt, bei dem die Membran 60 mit einem Zuggurtungsband 61 für den Rückgrad verbunden ist oder eine Einheit bildet. Mit dem Zuggurtungsband 61, werden die Wirbelkörper 65, zwischen denen beispielsweise eine nicht dargestellte künstliche Bandscheibe eingesetzt wurde, zusammengehalten. Das Zuggurtungsband 61 ist Bestandteil der Membran 60 und erfüllt auch die Funktion einer Membran. Für die lösbare Fixierung der Membran 60 sind im Zuggurtungsband 61 Löscher 62 vorgesehen, über die eine Befestigung mittels Schrauben 63 und der im Knochenmaterial fest und auf Dauer verankerten Muttern 64 erfolgt.

Die Bildung von Serum zwischen zwei Membranschichten wird auch dadurch verhindert, dass die beiden Membranschichten ohne Zwischenlage direkt miteinander lösbar verklebt werden.

im Beispiel gemäß Fig.4, der Zugang zur Operationsstelle 30 freigegeben wird. Mit durch den Stutzen 51 geführten Röhren und Instrumenten 52, wird der Fensterdeckel 43 hochgeklappt und der Eingriff im Bereich 30 vorgenommen. Während dessen wird der Luftdruck innerhalb der Membran 50 aufrechterhalten. Es können auch zwei Stutzen, einer für den Luftdruck und einer für die Instrumente vorgesehen werden. Nach Beendigung des Eingriffes wird der Fensterdeckel 43 zugeklappt und der Membraninnenraum erneut evakuiert.

Die Fixierung einer Membran erfolgt, soweit notwendig, je nach Anwendung durch Nähen, mit resorbierbaren oder beständigen Stapels oder mittels an Knochen 65 angebrachten Befestigungsplatten 64, Fig.6 oder dergleichen.

In der Fig.6 ist ein Beispiel gezeigt, bei dem die Membran 60 mit einem Zuggurtungsband 61 für den Rückgrad verbunden ist oder eine Einheit bildet. Mit dem Zuggurtungsband 61, werden die Wirbelkörper 65, zwischen denen beispielsweise eine nicht dargestellte künstliche Bandscheibe eingesetzt wurde, zusammengehalten. Das Zuggurtungsband 61 ist Bestandteil der Membran 60 und erfüllt auch die Funktion einer Membran. Für die lösbare Fixierung der Membran 60 sind im Zuggurtungsband 61 Löscher 62 vorgesehen, über die eine Befestigung mittels Schrauben 63 und der im Knochenmaterial fest und auf Dauer verankerten Muttern 64 erfolgt.

Die Bildung von Serum zwischen zwei Membranschichten wird auch dadurch verhindert, dass die beiden Membranschichten ohne Zwischenlage direkt miteinander lösbar verklebt werden. Der Einsatz mit einschichtigen Membranen erfolgt in ähnlicher Weise, wie bisher beschrieben. Im Fall der Fig.4 wird die einschichtige Membran z.B. mit den Organen/Gefäßen 31,32, ähnlich der Membranschicht 33, hochgehalten, womit die Operationsstelle 30 direkt zugänglich gemacht wird, da die zweite Membranschicht 34 fehlt. Die Membrankante 38 kann in diesem Fall lösbar mit den Wirbelkörpern 39, wie in Fig. 6 gezeigt, verbunden sein.

## Patentansprüche

1. Adhäsionsprophylaxe bestehend aus einer Membran aus körperverträglichem Material, die zwischen chirurgischer Eingriffstelle und angrenzenden Geweben zur Verhinderung einer Verklebung der Narbenstelle und Gewebe einlegbar ist, wobei die Adhäsionsprophylaxe (20;33-35) aus zwei übereinanderliegenden Membranen (21,22;33,34) besteht, die an einer Kante (38) miteinander verbunden sind, und daß zwischen den beiden übereinanderliegenden Membranen (21,22;33,34) eine mit diesen lösbar verbundene Zwischenlage (23;35) oder Zwischenschicht vorgesehen ist, die zwischen den Membranen herausziehbar ist, derart, dass bei einem Revisionseingriff eine sichere Trennung der beiden Membranschichten gewährleistet ist, wobei zur Hilfestellung bei der Freilegung der Eingriffstelle die beiden Membranschichten aufklappbar sind, und wobei die eine Membran (33) als Halterung zum Verschieben von Gewebe dient.

2. Adhäsionsprophylaxe, **dadurch gekennzeichnet, daß** die beiden übereinanderliegenden Membranen (21,22;33,34) unterschiedliche Eigenschaften haben.

3. Adhäsionsprophylaxe nach Anspruch 1 oder 2, **dadurch gekennzeichnet, daß** die Membran (60) ein Bestandteil eines Zuggurtungsbands (61) ist oder mit diesem verbunden ist.

## Claims

1. Adhesion prophylaxis consisting of a membrane of a body-compatible material which is insertable between a surgical incision site and adjacent tissue to prevent adhesion of the scar and tissue, said adhesion prophylaxis (20, 33-35) consisting of two superimposed membranes (21, 22; 33, 34) which are adhered to each other at an edge (38), wherein an intermediate sheet (23; 35) or intermediate layer is provided between the two superimposed membranes (21, 22; 33, 34) and detachably connected therewith, said intermediate sheet being configured to be pulled out between the membranes such that in a revision surgery a safe separation of the two membrane layers is ensured, wherein, in order to assist exposure of the incision site the two membrane layers are unfoldable, and wherein one membrane (33) serves as support for displacing tissue.

2. Adhesion prophylaxis, **characterized in that** the two superimposed membranes (21, 22; 33, 34) exhibit different properties.

3. Adhesion prophylaxis according to claim 1 or 2, **characterized in that** the membrane (60) forms part of a tension belt (61) or is connected thereto.

## Revendications

1. Prophylaxie d'adhésion constituée d'une membrane en matériau biocompatible qui peut être placée entre un site d'intervention chirurgicale et des tissus adjacents pour empêcher un collage du site cicatriciel et des tissus, dans laquelle la prophylaxie d'adhésion (20 ; 33-35) est constituée de deux membranes superposées (21, 22 ; 33, 34) qui sont reliées l'une à l'autre par un bord (38) et en ce qu'entre les deux membranes superposées (21, 22 ; 33, 34), une sous-couche (23 ; 35) ou une couche intermédiaire liée de façon amovible avec celles-ci, qui peut être retirée d'entre les membranes, est prévue de telle sorte que lors d'une intervention de révision, une séparation sûre des deux couches de membrane soit assurée, dans laquelle pour aider à l'exposition du site d'intervention, les deux couches de membrane sont repliables et dans laquelle une membrane (33) sert de support pour déplacer des tissus.

2. Prophylaxie d'adhésion, **caractérisée en ce que** les deux membranes superposées (21, 22 ; 33, 34) ont des propriétés différentes.

3. Prophylaxie d'adhésion selon la revendication 1 ou 2, **caractérisée en ce que** la membrane (60) est un composant d'une bande de sanglage (61) ou est reliée à celle-ci.
